# EUROPEAN PATENT SPECIFICATION

(11) **EP 0 343 874 B1**
(45) Date of publication and mention of the grant of the patent: **20.10.1993**
(21) Application number: 89305103.7
(22) Date of filing: 19.05.1989
(51) Int. Cl.: A61B 5/103, A61J 7/00

(54) **Therapeutic apparatus**
Therapeutisches Instrument
Appareil thérapeutique

(30) Priority: 23.05.1988 US 197592
(43) Date of publication of application: 29.11.1989
(73) Proprietor: Broselow, James B., Hickory North Carolina 28602 (US)
(72) Inventor: Broselow, James B., Hickory North Carolina 28602 (US)
(74) Representative: Atkinson, Ralph

(56) References cited:
- EP-A- 0 220 860
- FR-A- 1 453 008
- US-A- 4 345 541

## Description

This invention relates to therapeutic apparatus which can be used in emergency situations where it is necessary to determine with accuracy and with speed a variable of the treatment.

Perhaps the most obvious variable is that of drug or medicine dosage. It is quite usual for drug or medicine dosage to be determined by the age of the patient (particularly if the patient is an infant or child) and this is almost the only method used in the case of over-the-counter (non-prescription) medicines but this is a very inaccurate method and would be totally inappropriate in the administration of toxic drugs, for instance in chemotherapy. Besides, the age of the patient is not always readily determinable in emergencies or in situations where language and/or illiteracy may be a problem.

In medical practice, drug dosages have been based historically on the body weight of the patient but there are many situations where this is impracticable. For instance, in village communities or developing countries weighing apparatus may not be available, and in other communities there may be no one with the ability to use appropriate weighing apparatus.

Even in situations where trained medical personnel are available there are problems with the body weight method of controlling drug dosage. The physician must first estimate the weight of the patient (it is not always practicable to weigh the patient especially in emergencies), then either consult tables to determine the appropriate dosage, or determine the dosage by memory, and then multiply that dosage by the estimated weight.

The possibility of error is present and is increased by the pressure of time and circumstances which often attend emergency medical treatment. On the other hand, as medical treatment has become more complex, drug dosages have become more critical, and therefore errors could have very dangerous, and even lethal results.

Another example of a therapeutic treatment variable is the tube size of an endotracheal tube, which has to be introduced through the epiglottis of the patient. On the one hand the tube must not be too large, or it will damage the throat tissues, and on the other hand, if it is too small, it will leave a clearance which could vitiate the treatment entirely. Clearly the correct tube size is related to the size of the patient. Another patient-size related variable is that of tube length in the case of an endotracheal tube.

It will be appreciated therefore that in therapeutic treatment, there is a range of variables measurable in physical quantities or dimensions, and it is a fact that estimating the weight of the patient is the most commonly used method of assessing the variables.

However, it has been recognised that in many cases, weight is an inaccurate and inappropriate basis on which to determine physical treatment values such as drug dosages, tube lengths and sizes, medical equipment settings and like patient-related treatment and apparatus. Despite this, weight remains the most commonly practiced way of determining these values. In many cases, length is a much more appropriate way to determine many physical treatment values. For example, the length of an endotracheal tube is very closely related to the overall length of a patient, whether that patient is underweight, overweight or of an ideal weight. Likewise, it is now known that an obese patient weighing significantly more than a patient of normal weight will certainly not need an increase in the dosage of most drugs proportional to his weight and, in fact, can be dosed to toxic levels in this way. This is because many drugs distribute only in the lean body tissue.

The present invention provides apparatus enabling accurate and swift determination of a correct therapeutic treatment to be administered, which is based on the length of the patient.

Specification EP-A-0 220 860 J. B. BROSELOW describes a measuring tape used for measuring the heel-to-crown height of a patient; but instead of bearing conventional measurement markings, the tape is divided into zones, each corresponding to an increment of length of the patient and each zone bears printed information concerning, for example, drug dosages, defibrillation settings and tube sizes appropriate to the treatment of a patient of a length corresponding to that increment.

In order to use the tape disclosed in EP-A-0 220 860, the paediatrician, or whoever is administering treatment, must read the tape carefully and then measure out the correct drug dosage or select the correct apparatus size in accordance with the instructions printed on the tape. The problem which can arise in certain emergency situations is that the person required to use the tape may not have the required literacy skills. To express this another way, the literacy demanded of the user by the tape disclosed in the cited European Specification is such that many people simply could not use the tape.

According to this invention, apparatus for selection of emergency medical equipment and drug dosages for therapeutic treatment of a patient comprises: a measuring tape for measuring the heel-to-crown height of a patient, said measuring tape having coded zones along its effective length distinguished from each other by marking identifiable without literacy, and a treatment dispenser having a plurality of coded sectors corresponding to the coded zones on the tape, each sector defining a medicament dosage or medical equipment of a specific size, the sectors being distinguished from each other by marking of the same kind as that which distinguishes the zones of the measuring tape from each other, so that each sector of the dispenser is co-related with one of the zones of the tape, whereby a dosage or apparatus obtained from that sector of the dispenser is co-related to the heel-to-crown height of a patient as measured by the tape.

The background to the invention has been explained with reference to medical practice, but it is to be understood that it is equally applicable to veterinary practice and therefore the term "patient" is to be construed as including animals as well as humans. For simplicity, however, the specific examples quoted herein relate to the treatment of humans.

Also the term "dispenser" is to be broadly construed. A very simple dispenser may comprise a cup or spoon for administering liquids orally; a more sophisticated dispenser is a droplet type dispenser which can be used to administer very small and critical quantities of drugs, and another form of dispenser comprises a series of compartments, each containing one or more items of apparatus (e.g. endotracheal tubes).

A simple and very effective coding is provided by colour coding, but other codes such as cross hatching; or simple easily distinguished illustrations may be employed.

To take a simple illustration, the measuring tape may be divided into differently coloured increments of length (e.g. red; green; yellow; blue; white) and a cup-type medicine dispenser for use with the tape marked with levels shown as red; green; yellow; blue; white. Therefore, by simply measuring the patient and filling the dispenser to the colour level corresponding to the colour on the tape read off from the patient's height, it is ensured the medicine dose is co-related to the height of the patient. However, quite critical drug dosages can also be dispensed by the same method using a more sophisticated, but similarly coded dispenser.

Taking another illustration, in a hospital emergency or casualty ward, the dispenser may comprise a set of containers (e.g. drawers) each containing a set of apparatus likely to be needed in emergency treatment, but with different sizes of some or all of the items in each container. When a patient is to receive treatment the appropriate colour code is read off by measuring the patient's height and then the container of that colour is selected. The user is then presented with a set of apparatus all of the correct sizes appropriate to the patient's length.

Obvious advantages of the invention are that the selection of the correct variable can be made very quickly, (since it is only necessary to measure the patient's length) and that the selection can be made by a relatively untrained staff, certainly by illiterate personnel (or personnel illiterate in the language of any printed instructions) and without any calculations. The possibility of error is greatly reduced and the variable selected according to heel to crown height which is usually a more useful guide than estimated body weight.

The invention will be more clearly understood from the following description of certain specific embodiments of the invention which are described by way of examples only, and with reference to the accompanying drawings in which ; -
Figure 1 is a perspective view of one embodiment of a measuring tape according to the present invention,
Figure 2 is a perspective view illustrating the manner of using the measuring tape illustrated in Figure 1,
Figure 3 is a side elevational view of the measuring tape mounted for use on the side of a stretcher,
Figure 4 is a side elevational view of the measuring tape affixed to the side of a stretcher in an extended form,
Figure 5 is a plan view of the measuring tap in accordance with this embodiment,
Figure 6 is an elevational view of the cup type dispenser for liquids,
Figure 7 is a view similar to Figure 6, but showing a different method of coding a cup type dispenser,
Figure 8 is a perspective view of a drawer type dispenser, and
Figure 9 is a plan view of an alternative form of measuring tape showing another method of coding.

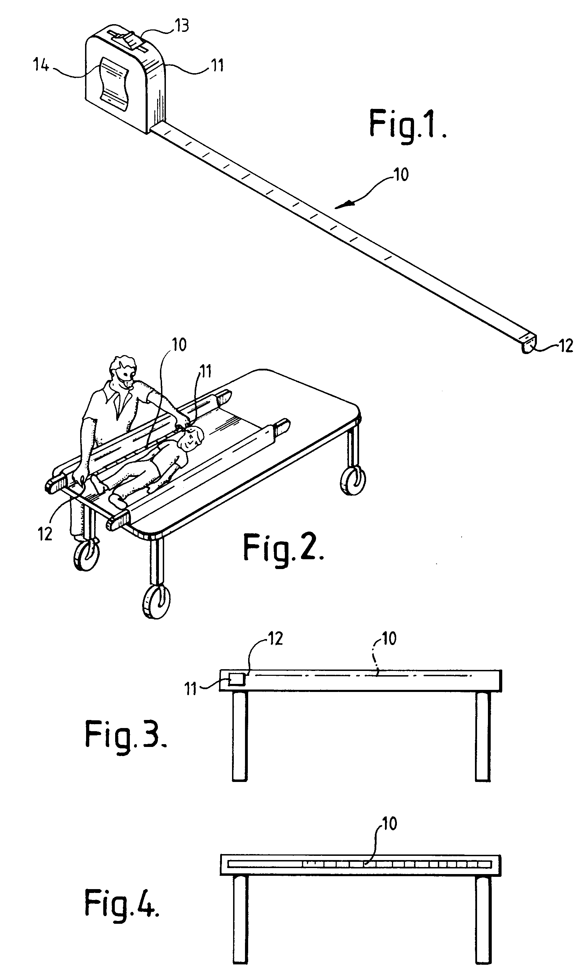

A measuring tape according to a first embodiment of the invention is shown in Figure 1 and indicated by the reference number 10. The tape 10 can be constructed with various differing physical characteristics. Preferably, it comprises a steel type tape having a cupped cross section which provides increased rigidity to the tape and at the same time allows flexibility when needed. According to the embodiment shown in Figure 1, the tape is retractably mounted inside an enclosure 11. One end of the tape is connected to a spring return mechanism (not shown) located within the enclosure 11. The tape is used by withdrawing it from the enclosure 11 by means of a pull tab 12 at the free end of the tape. To retract the end of the tape, a button 13 on the enclosure 11 is pressed, and this causes the tape 10 to retract into the enclosure 11. A belt clip 14 is provided on one side of the enclosure 11 so that the enclosure can be secured to the user's belt or to a pocket.

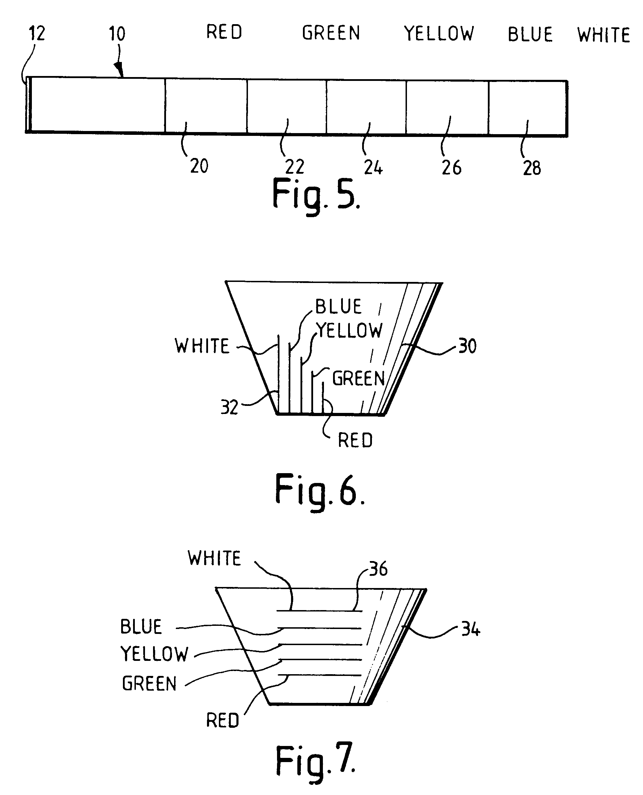

Turning now to Figure 5, there is illustrated the manner in which the measuring tape 10 is divided into a series of length increments 20, 22, 24, 26 and 28. Figure 5 also illustrates how these length increments are colour coded, that is to say each increment of the measuring tape has a distinctive colour, in this case, the colour coding being as follows :-
- 20: Red
- 22: Green
- 24: Yellow
- 26: Blue
- 28: White
The colour coded length measurements are so arranged, that if the tape is used to measure the heel to crown height of a patient, that measured height which will be read off as a colour code, will in fact be co-related to the ideal or lean body weight of the patient, although the tape itself will give no direct weight measurement. Therefore, the measuring tape 10 automatically takes into account that for example, drug dosage does not increase in direct proportion to an increase in weight of the patient. This is desirable, since most emergency drug dosages are based on "lean" body weight, because drugs do not distribute into fatty tissue at the same rate or to the same extent as into organ and muscle tissue during the time frame of emergency treatment.

In Figure 6, there is illustrated a simple transparent plastics cup 30, which can be used for the administration of medicines to a patient. It will be observed, that a series of vertical lines 32 is provided on the wall of the cup 30, and these lines may in fact be etched into the wall of the cup. Moreover, the vertical lines 32 are of differing lengths, and are colour coded, to the same as those used for the length increments on the measuring tape 10. The arrangement of the vertical lines 32, is such that for a given type of medicine, a correct dose for a patient having a height measured into the red increment 20 on the tape 10, would be given by filling the cup 30 to the top of the red vertical line. Similarly, a correct dosage of the medicine would be administered to a patient measured into say the blue zone of the measuring tape 10, by filling the cup 20 up to the top of the blue vertical line.

It will be appreciated therefore, that there is a direct co-relation between the measured heel to crown height of the patient, and the medicine which is administered. This co-relation is obtained without any calculation, and without the necessity for the reading off of numerical values on the measuring tape. Moreover, it is not necessary for the person administering the medicine to be literate since no instructions have to be read. Hence, the apparatus comprising the measuring tape 10 and the dispensing cup 30 can be used in situations where it is not practicable to have medically trained supervision. For instance, in areas remote from medical personnel, untrained people, such as the parent of a child, will be able to give the correct dosage of a medicine over a length of time during which the child will be growing, by simply measuring the height of the child using the tape 10, and then selecting the appropriate fill of the cup as given by the corresponding colour coding on the cup.

In Figure 7, there is illustrated a cup 34 which is very similar to the cup 30 illustrated in Figure 6, excepting that the vertical colour coded lines 32 are replaced by horizontal colour coded lines 36.

Some medicines and drugs are administered by more sophisticated dispensers such as droplet dispensers, or hyperdermic needles. However, it will be appreciated that these more sophisticated types of dispenser could have colour coding applied thereto corresponding to the colour coding of a measuring tape of the kind illustrated at 10. For instance, where the drug is pre-packed in dispensers of differing sizes, the different sizes of dispenser could be colour coded in accordance with the colour code on the measuring tape. Alternatively where the dispenser has a transparent zone through which the drug can be observed, there may be a colour coding on the transparent zone which will enable the person administering the drug to use a quantity dictated in accordance with the colour coding. Therefore, even in quite sophisticated medical practice, the tape measure 10 can be used in association with a correspondingly colour coded dispenser or set of dispensers in order to give a dosage which is related to the height of the patient, and thereby indirectly related to the ideal weight of the patient.

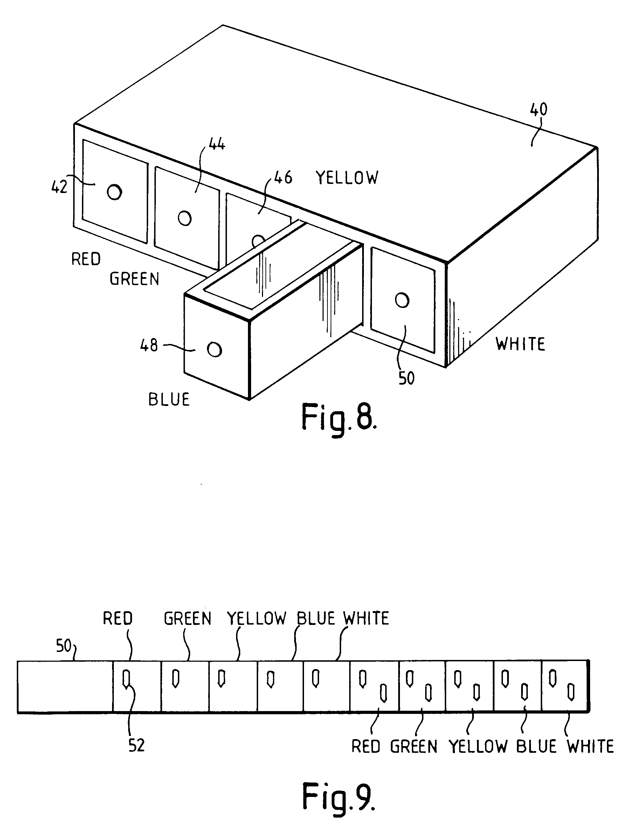

Turning now to Figure 8, there is illustrated a dispenser, which is intended to be used in an emergency or casualty ward of a hospital, and which in effect comprises a container 40 comprising a series of drawers 42; 44; 46; 48 and 50. Figure 8 illustrates how the fronts of these drawers are coloured according to a similar colour coding scheme as that applied to the measuring tape 10, that is to say the drawer 42 is coloured red, the drawer 44 is coloured green and so on.

In use, each of these drawers 42 to 50 contains either a single piece of medical or surgical apparatus, such as an endotracheal tube, or it may contain a kit of items of apparatus, including pre packed drugs. The significance of the arrangement is that each drawer contains an apparatus or kit of pieces of apparatus, which are of the correct values in terms of dosage or size, for the treatment of a patient the height of which has been read off according to the colour coding on the measuring tape 10.

One of the problems of an emergency or casualty ward, is that treatment generally has to be initiated under pressure. On the other hand, the size of certain pieces of equipment, for instance the diameter of an endotracheal tube can be quite critical to the success of the treatment which is some cases may be vital. However, by simply measuring the heel to crown height of the patient, reading off the appropriate colour coding, and then selecting the correspondingly colour code drawer, it is ensured that the apparatus which is used is correct for the patient having that height. Again therefore, it will be appreciated that there is a great saving in time and stress on the staff in the emergency situation, and some guarantee that the correct treatment will be applied.

In some instances, simple colour coding by itself may not be sufficient, since obviously it is best if the colour coding can rely on only primary colours, and more than a few length increments may be needed. Figure 9 illustrates an alternative measuring tape 50 having a series of ten length increment zones. It will be observed from Figure 9 that the zones are coloured in the sequence : red; green; yellow; blue; white; red; green; yellow; blue; white. However, the first five zones each bear the representation (diagrammatic) of a single dispenser 52, whereas the second set of five zones each has the representation of two dispensers. This is intended to indicate to a user that, if the measured length of a patient is in the second set of five colour coded zones, the appropriate dosage will be twice that which will be given if the measured length were in one of the first five colour coded zones. This therefore illustrates a method whereby the colour coding may be supplemented by an additional coding method to increase the number of length increments which are available on the measuring tape. It will be appreciated therefore that colour coding is not the only method by which the dispenser could be coded, for instance simple symbol coding could be employed.

Referring back now to Figure 2, the tape 10 or 50 can be used as a conventional measuring tape. That is, the enclosure 11 is placed at one end of the patient and the measuring tape 10 is extended so that the put tab 12 of the measuring tape is at the other end of the patient. Then the coding is read directly off the tape 10 as previously described.

As is shown in Figure 3, the enclosure 11 can be mounted at the head or foot end of a stretcher. When a patient is placed on a stretcher, a measurement can be taken by extending the measuring tape 10 along the length of the stretcher to the other end of the patient's body. Another variation of the invention is shown in Figure 4, where a measuring tape 10 is fixed along the length of a stretcher, so that a reading can be taken directly without manipulating the tape at all. It is only necessary to make sure that one of the patient's body is adjacent to one end of the tape.

## Claims

1. Apparatus for selection of emergency medical equipment and drug dosages for therapeutic treatment of a patient comprising a measuring tape (10) for measuring the heel-to-crown height of a patient, said measuring tape having coded zones along its effective length distinguished from each other by marking identifiable without literacy, and a treatment dispenser (30, 34, 40) having a plurality of coded sectors corresponding to the coded zones on the tape, each sector defining a medicament dosage or medical equipment of a specific size, the sectors being distinguished from each other by marking of the same kind as that which distinguishes the zones of the measuring tape from each other, so that each sector of the dispenser is co-related with one of the zones of the tape, whereby a dosage or apparatus obtained from that sector of the dispenser is co-related to the heel-to-crown height of a patient as measured by the tape.

2. Apparatus for the selection of emergency medical equipment and drug dosages for therapeutic treatment of a patient according to claim 1, wherein said dispenser (30, 34) comprises a receptacle for administering liquids orally, the sectors being defined by markings, each marking indicating a liquid capacity of a correct dosage value indicated by the corresponding marking on the measuring tape.

3. Apparatus for the selection of emergency medical equipment and drug dosages for therapeutic treatment of a patient according to claim 2, wherein said dispenser comprises a droplet-type dispenser, which can be used to administer very small and critical quantities of drugs.

4. Apparatus for the selection of emergency medical equipment and drug dosages for therapeutic treatment of a patient as claimed in claim 1, wherein each of the sectors comprises an accessible compartment containing at least one pre-prepared dosage of a medicament, and for at least one item of medical apparatus of a specific size indicated by the corresponding marking on the measuring tape.

5. Apparatus for the selection of emergency medical equipment and drug dosages for therapeutic treatment of a patient according to claim 1, wherein the marking of both the measuring tape and the dispenser comprises colour coding.

## Patentansprüche

1. Apparat zum Auswählen von medizinischer Notfallausrüstung und Medikamentendosierungen für die therapeutische Behandlung eines Patienten, bestehend aus einem Maßband (10) zum Messen der gesamten Körpergröße eines Patienten, wobei das erwähnte Maßband codierte Zonen entlang seiner gesamten Länge hat, die voneinander unterschieden werden durch erkennbares Markieren ohne Beschriftung, sowie einem Behandlungsspender (30, 34, 40), der eine Vielzahl von codierten Sektoren hat, die den codierten Zonen auf dem Band entsprechen, wobei jeder Sektor eine Medikamentendosierung oder medizinische Ausrüstung einer bestimmten Größe definiert, wobei die Sektoren voneinander unterschieden werden durch Markierungen derselben Art, wie jene, die die Zonen auf dem Maßband unterscheiden, so daß jeder Sektor des Spenders mit einer der Zonen des Bandes in Beziehung steht, wobei eine Dosierung oder ein Apparat, der sich aus diesem Abschnitt des Spenders ergibt, in Beziehung steht mit der gesamten Körpergröße eines Patienten, so wie sie vom Band gemessen wird.

2. Apparat zum Auswählen von medizinischer Notfallausrüstung und Medikamentendosierung zur therapeutischen Behandlung eines Patienten gemäß Anspruch 1, wo der erwähnte Spender (30, 34) einen Behälter zum oralen Verabreichen von Flüssigkeiten umfaßt, die Sektoren durch Markierungen bestimmt sind und jede Markierung eine Flüssigkeitskapazität einer korrekten Dosierung anzeigt, die angezeigt wird durch die entsprechende Markierung auf dem Maßband.

3. Apparat zum Auswählen von medizinischer Notfallausrüstung und Medikamentendosierung zur therapeutischen Behandlung eines Patienten gemäß Anspruch 2, wobei der erwähnte Spender einen Tropfspender beinhaltet, der benutzt werden kann zum verabreichen sehr kleiner und gefährlicher Mengen von Medikamenten.

4. Apparat zum Auswählen von medizinischer Notfallausrüstung und Medikamentendosierung zur therapeutischen Behandlung eines Patienten wie in Anspruch 1 beansprucht, wobei jeder der Sektoren ein zugängliches Containerschubfach umfaßt, mindestens eine vorher vorherbereitete Dosierung eines Medikaments, und für mindestens ein Stück eines medizinischen Apparats einer speziellen Größe, die angezeigt wird durch die entsprechende Markierung auf dem Maßband.

5. Apparat zum Auswählen von medizinischer Notfallausrüstung und Medikamentendosierung zur therapeutischen Behandlung eines Patienten gemäß Anspruch 1, wo sowohl die Markierung des Maßbandes als auch des Spenders einen Farbcode beinhaltet.

## Revendications

1. Appareil pour sélectionner des équipements médicaux d'urgence et des dosages de médicaments en vue du traitement thérapeutique d'un patient, comportant un ruban de mesure (10) destiné à mesurer la taille du patient des talons au sommet de la tête, ledit ruban de mesure présentant dans le sens de sa longueur effective des zones codées, distinguées les unes des autres par un marquage identifiable par un analphabète, et un distributeur de traitement (30, 34, 40) pourvu de plusieurs secteurs codés qui correspondent aux zones codées prévues sur le ruban, chaque secteur définissant un dosage de médicament ou un équipement médical de dimensions spécifiques les secteurs étant distingués les uns des autres par un marquage de même nature que celui qui distingue les unes des autres les zones du ruban de mesure, afin que chaque secteur du distributeur soit en corrélation avec l'une des zones du ruban, pour qu'ainsi un dosage ou un instrument obtenu à partir de ce secteur du distributeur soit en corrélation avec la taille des talons au sommet de la tête du patient mesuré à l'aide du ruban.

2. Appareil pour sélectionner des équipements médicaux d'urgence et des dosages de médicaments en vue du traitement thérapeutique d'un patient, selon la revendication 1, dans lequel ledit distributeur (30, 34) comporte un récipient destiné à administrer des liquides par voie orale, les secteurs étant définis par des marquages dont chacun indique un volume de liquide correspondant à une valeur de dosage correcte indiquée par le marquage correspondant prévu sur le ruban de mesure.

3. Appareil pour sélectionner des équipements médicaux d'urgence et des dosages de médicaments en vue du traitement thérapeutique d'un patient, selon la revendication 2, dans lequel ledit distributeur comporte un distributeur du type compte-gouttes susceptible d'être utilisé pour administrer des quantités de médicament très faibles et critiques.

4. Appareil pour sélectionner des équipements médicaux d'urgence et des dosages de médicaments en vue du traitement thérapeutique d'un patient, selon la revendication 1, dans lequel chacun des secteurs comporte un compartiment accessible contenant au moins un dosage préparé préalablement d'un médicament, et au moins un élément d'un instrument médical ayant des dimensions spécifiques indiquées par le marquage correspondant prévu sur le ruban de mesure.

5. Appareil pour sélectionner des équipements médicaux d'urgence et des dosages de médicaments en vue du traitement thérapeutique d'un patient, selon la revendication 1 dans lequel le marquage aussi bien du ruban de mesure que du distributeur comporte un codage de couleur.
